# EUROPEAN PATENT APPLICATION

(11) **EP 4 653 847 A2**
(43) Date of publication of application: **26.11.2025**
(21) Application number: 25173628.6
(22) Date of filing: 30.04.2025
(51) Int. Cl.: G01N 21/45, G01N 33/483, H01S 5/0683, H01S 5/11, H01S 5/187

(54) **SYSTEM FOR MONITORING AND INTERFEROMETRY BASED ON PCSELS EMITTING IN TWO DIRECTIONS**

(30) Priority: 02.05.2024 US 202418653268
(71) Applicant: II-VI Delaware, Inc., Wilmington, DE 19890 (US)
(72) Inventor: TATARCZAK, Anna, Wilmington, 19890 (US)
(74) Representative: Schmidt, Christian

(57) **Abstract**

Embodiments of the present disclosure are for a system comprising a PCSEL generating a first and a second light beam. The physical emission direction of the first light beam is at an angle of approximately 180 degrees to a physical emission direction of the second light beam. The system comprises a driver configured to receive an electrical input and generate a modulation signal coupled to the PCSEL. A monitoring photodetector is coupled to the second light beam, to generate an electrical monitoring output signal to monitor the first and the second light beam.

## Description

### TECHNICAL FIELD

The present disclosure generally relates to a system for monitoring and interferometry based on PCSELs emitting in two directions.

### BACKGROUND

Aspects of the present disclosure relate to a system for monitoring and interferometry based on PCSELs emitting in two directions. Various issues may exist with conventional solutions for systems for monitoring and interferometry based on PCSELs emitting in two directions. In this regard, conventional systems and methods for coherent receivers may be costly, cumbersome, and/or inefficient.

Limitations and disadvantages of conventional systems and methods will become apparent to one of skill in the art, through comparison of such approaches with some aspects of the present methods and systems set forth in the remainder of this disclosure with reference to the drawings.

### BRIEF SUMMARY OF THE DISCLOSURE

Shown in and/or described in connection with at least one of the figures, and set forth more completely in the claims is a system for monitoring and interferometry based on PCSELs emitting in two directions.

These and other advantages, aspects and novel features of the present disclosure, as well as details of illustrated embodiments thereof, will be more fully understood from the following description and drawings.

### BRIEF DESCRIPTION OF THE DRAWINGS

The various features and advantages of the present disclosure may be more readily understood with reference to the following detailed description taken in conjunction with the accompanying drawings, wherein like reference numerals designate like structural elements.
FIG. 1 is a block diagram illustrating a system, according to some embodiments of the present disclosure.
FIG. 1A is a diagram further illustrating the system of FIG. 1, according to some embodiments of the present disclosure.
FIG. 2 is a block diagram illustrating an interferometry system, according to some embodiments of the present disclosure.
FIG. 2A is a diagram further illustrating the system of FIG. 2, according to some embodiments of the present disclosure.

### DESCRIPTION

The following discussion provides various examples of devices and of manufacturing devices. Such examples are non-limiting, and the scope of the appended claims should not be limited to the particular examples disclosed. In the following discussion, the terms "example" and "e.g." are non-limiting.

The figures illustrate the general manner of construction, and descriptions and details of well-known features and techniques may be omitted to avoid unnecessarily obscuring the present disclosure. In addition, elements in the drawing figures are not necessarily drawn to scale. For example, the dimensions of some of the elements in the figures may be exaggerated relative to other elements to help improve understanding of the examples discussed in the present disclosure. The same reference numerals in different figures denote the same elements.

The term "or" means any one or more of the items in the list joined by "or". As an example, "x or y" means any element of the three-element set {(x), (y), (x, y)}. As another example, "x, y, or z" means any element of the seven-element set {(x), (y), (z), (x, y), (x, z), (y, z), (x, y, z)}.

The terms "comprises," "comprising," "includes," and/or "including," are "open ended" terms and specify the presence of stated features, but do not preclude the presence or addition of one or more other features.

The terms "first," "second," etc. may be used herein to describe various elements, and these elements should not be limited by these terms. These terms are only used to distinguish one element from another. Thus, for example, a first element discussed in this disclosure could be termed a second element without departing from the teachings of the present disclosure.

Unless specified otherwise, the term "coupled" may be used to describe two elements directly contacting each other or describe two elements indirectly connected by one or more other elements. For example, if element A is coupled to element B, then element A can be directly contacting element B or indirectly connected to element B by an intervening element C. Similarly, the terms "over" or "on" may be used to describe two elements directly contacting each other or describe two elements indirectly connected by one or more other elements.

Embodiments of the present disclosure may comprise a system, the system comprising a PCSEL generating a first and a second light beam. In accordance with various embodiments, a physical emission direction of the first light beam may be at an angle of approximately 180 degrees to a physical emission direction of the second light beam. Embodiments may also comprise a driver configured to receive an electrical input and generate a modulation signal coupled to the PCSEL. The modulation signal may control the PCSEL. The modulation signal may be pulsed or continuous wavelength, for example. Embodiments may also comprise a monitoring photodetector coupled to the second light beam, configured to generate an electrical monitoring output signal configured to monitor the first and the second light beam.

In accordance with various embodiments, the first light beam and the second light beam may be substantially identical. In accordance with various embodiments, the second light beam may carry a fraction of the power of first light beam, or vice versa. In accordance with various embodiments, the modulation signal may be configured to control a frequency and/or an output power of the PCSEL. In accordance with various embodiments, the system may comprise a feedback control unit coupled to the monitoring photodetector and configured to generate the electrical input at the driver, based on the electrical monitoring output signal. In accordance with various embodiments, the feedback control unit may be configured to maintain a desirable output power and/or output frequency for the first and the second light beam generated at the PCSEL.

Embodiments of the present disclosure may also comprise an interferometry system, the system comprising a PCSEL generating a first and a second light beam. In accordance with various embodiments, a physical emission direction of the first light beam may be at an angle of approximately 180 degrees to a physical emission direction of the second light beam.

Embodiments may also comprise a specimen configured to receive the first light beam. The specimen may be, for example, living tissue and is not part of the system itself but the system may be used to infer properties of the specimen by using techniques of this disclosure. Embodiments may also comprise a probe coupled to the specimen, configured to receive light comprising at least a portion of the first light beam after it has traversed the specimen. Embodiments may also comprise a beam combiner configured to receive and combine the second light beam and the received light into a combined beam. Embodiments may also comprise a receiver configured to receive the combined beam, generate an electrical signal based on the combined beam and process the electrical signal. For example, interference contributions, phase, and amplitude information of the combined beam may be utilized.

In accordance with various embodiments, the specimen may be biological tissue. In accordance with various embodiments, the first light beam may be coupled to the specimen using optical fiber. In accordance with various embodiments, the first light beam may be couple to the specimen using free space transmission, which may use optical elements such as lenses. In accordance with various embodiments, the first light beam entering the specimen may be scattered and reflected within the specimen to generate the received light at the probe or at the receiver. In accordance with various embodiments, the beam combiner interferes the second light beam with the received light by combining the two beams.

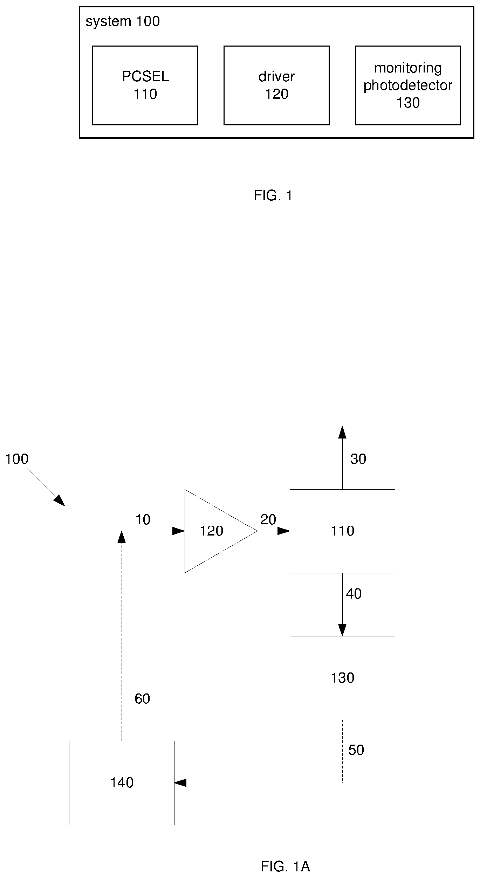

Referring now to FIG. 1, FIG. 1 is a block diagram that describes a system 100, according to some embodiments of the present disclosure. In some embodiments, the system 100 may comprise a PCSEL 110 generating a first and a second light beam. The system 100 may also include a driver 120 configured to receive an electrical input and generate a modulation signal coupled to the PCSEL 110, for controlling and/or driving the PCSEL 110. The system 100 may also include a monitoring photodetector 130 coupled to the second light beam, configured to generate an electrical monitoring output signal configured to monitor the first and the second light beam. A physical emission direction of the first light beam may be at an angle of approximately 180 degrees to a physical emission direction of the second light beam.

In some embodiments, the first light beam and the second light beam may be substantially identical. In some embodiments, the modulation signal may be configured to control a frequency and/or an output power of the PCSEL 110. In some embodiments, the system 100 may also include a feedback control unit coupled to the monitoring photodetector 130 and configured to generate the electrical input at the driver 120, based on the electrical monitoring output signal. In some embodiments, the feedback control unit may be configured to maintain a desirable output power and/or output frequency for the first and the second light beam generated at the PCSEL 110.

FIG. 1A is a diagram further illustrating the system of FIG. 1, according to some embodiments of the present disclosure. The system 100 may comprise a PCSEL 110 generating a first light beam 30 and a second light beam 40. The system 100 may also include a driver 120 configured to receive an electrical input 10 and generate a modulation signal 20 coupled to the PCSEL 110, for driving and/or controlling the PCSEL 110. The system 100 may also include a monitoring photodetector 130 coupled to the second light beam 40, configured to generate an electrical monitoring output signal 50 configured to monitor the first light beam 30 and the second light beam 40. A physical emission direction of the first light beam 30 may be at an angle of approximately 180 degrees to a physical emission direction of the second light beam 40.

In some embodiments, the first light beam 30 and the second light beam 40 may be substantially identical. In some embodiments, the modulation signal 20 may be configured to control a frequency and/or an output power of the PCSEL 110. In some embodiments, the system 100 may also include a feedback control unit 140 coupled to the monitoring photodetector 130 and configured to generate the electrical input 10 (via 60) at the driver 120, based on the electrical monitoring output signal 50. In some embodiments, the feedback control unit 140 may be configured to maintain a desirable output power and/or output frequency for the first light beam 30 and the second light beam 40 generated at the PCSEL 110.

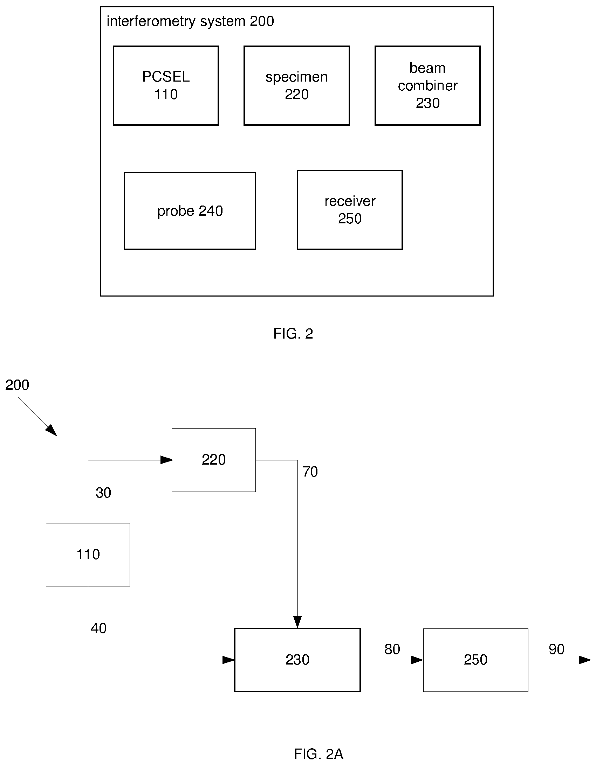

FIG. 2 is a block diagram that describes an interferometry system 200, according to some embodiments of the present disclosure. In some embodiments, the interferometry system 200 may include a PCSEL 110 generating a first and a second light beam, a specimen 220 configured to receive the first light beam, and a beam combiner 230 configured to receive and combine the second light beam and the received light into a combined beam. The interferometry system 200 may also include a probe 240 coupled to the specimen 220, configured to receive light. The interferometry system 200 may also include a receiver 250 configured to receive the combined beam, generate an electrical signal from the combined beam and process the electrical signal. In accordance with various embodiments, the electrical signal may be based on phase and amplitude information contained in the interference components of the combined beam, for example.A physical emission direction of the first light beam may be at an angle of approximately 180 degrees to a physical emission direction of the second light beam. The probe 240 may include at least a portion 242 of the first light beam after it traversed the specimen 220.

FIG. 2A is a diagram further illustrating the system of FIG. 2, according to some embodiments of the present disclosure. In some embodiments, the interferometry system 200 may include a PCSEL 110 generating a first light beam 30 and a second light beam 40, a specimen 220 configured to receive the first light beam 30, and a beam combiner 230 configured to receive and combine the second light beam 40 and the received light 70 into a combined beam 80. The interferometry system 200 may also include a probe 240 (not shown in FIG. 2A) coupled to the specimen 220, configured to receive light 70. The interferometry system 200 may also include a receiver 250 configured to receive the combined beam 80, generate an electrical signal 90 from the combined beam 80 and in some instances process the electrical signal 90 before outputting it. A physical emission direction of the first light beam 30 may be at an angle of approximately 180 degrees to a physical emission direction of the second light beam 40. The light received at probe 240 may include at least a portion of the first light beam 30 after it traversed the specimen 220.

In some embodiments, the specimen 220 may be biological tissue. For example, the first light beam 30 may be applied to a human or an animal to determine some medical parameters. In some embodiments, the first light beam 30 may be coupled to the specimen 220 using optical fiber. In some embodiments, the first light beam entering the specimen 220 may be scattered and reflected within the specimen 220 to generate the received light 70 at the probe 240. In some embodiments, the beam combiner 230 may interfere the second light beam 40 with the received light 70, by combining the beams.

The present disclosure includes reference to certain examples, however, it will be understood by those skilled in the art that various changes may be made and equivalents may be substituted without departing from the scope of the disclosure. In addition, modifications may be made to the disclosed examples without departing from the scope of the present disclosure. Therefore, it is intended that the present disclosure not be limited to the examples disclosed, but that the disclosure will include all examples falling within the scope of the appended claims.

## Claims

1. A system, said system comprising:
a PCSEL generating a first and a second light beam, wherein a physical emission direction of said first light beam is at an angle of approximately 180 degrees to a physical emission direction of said second light beam;
a driver configured to receive an electrical input and generate a modulation signal coupled to said PCSEL, for controlling said PCSEL;
a monitoring photodetector coupled to said second light beam, configured to generate an electrical monitoring output signal configured to monitor said first and said second light beam.

2. The system of claim 1, wherein said first light beam and said second light beam are substantially identical, or said second light beam may be generated by splitting off a fraction of power of said first light beam.

3. The system of claim 1, wherein said modulation signal is configured to control a frequency and/or an output power of said PCSEL.

4. The system of claim 1, further comprising a feedback control unit coupled to said monitoring photodetector and configured to generate said electrical input at said driver, based on said electrical monitoring output signal.

5. The system of claim 4, wherein said feedback control unit is configured to maintain a desirable output power and/or output frequency for said first and said second light beam generated at said PCSEL.

6. An interferometry system, said system comprising:
a PCSEL generating a first and a second light beam, wherein a physical emission direction of said first light beam is at an angle of approximately 180 degrees to a physical emission direction of said second light beam;
wherein a specimen configured to receive said first light beam is coupled to said system;
a probe coupled to said specimen, configured to receive light comprising at least a portion of said first light beam after it has traversed said specimen;
a beam combiner configured to receive and combine said second light beam and said received light into a combined beam;
a receiver configured to receive said combined beam, generate an electrical signal from said combined beam and process said electrical signal.

7. The system of claim 6, wherein said specimen is biological tissue.

8. The system of claim 6, wherein said first light beam is coupled to said specimen using optical fiber.

9. The system of claim 6, wherein said first light beam entering said specimen is scattered and reflected within said specimen to generate said received light at said probe or said receiver.

10. The system of claim 6, wherein said beam combiner interferes said second light beam with said received light.

11. The system of claim 6, wherein said receiver comprises at least one photodiode to receive said combined beam and generates said electrical signal from phase and/or amplitude information combined in interference signal components of said combined beam.
